# EUROPEAN PATENT SPECIFICATION

(11) **EP 2 756 840 B1**
(45) Date of publication and mention of the grant of the patent: **19.08.2020**
(21) Application number: 12832375.5
(22) Date of filing: 13.09.2012
(51) Int. Cl.: A61K 9/14, A61K 9/72, A61K 31/4418, A61K 47/26, A61P 11/00

(54) **PHARMACEUTICAL COMPOSITION FOR INHALATION**
PHARMAZEUTISCHE ZUSAMMENSETZUNGEN ZUR INHALATION
COMPOSITION PHARMACEUTIQUE DESTINÉE À ÊTRE INHALÉE

(30) Priority: 14.09.2011 JP 2011200150
(43) Date of publication of application: 23.07.2014
(73) Proprietor: Shionogi & Co., Ltd., Osaka-shi, Osaka 541-0045 (JP); Shizuoka Prefectural University Corporation, Shizuoka-shi, Shizuoka 422-8526 (JP)
(72) Inventor: ONOUE, Satomi, Shizuoka-shi Shizuoka 422-8526 (JP); YAMADA, Shizuo, Shizuoka-shi Shizuoka 422-8526 (JP)
(74) Representative: Boult Wade Tennant LLP
(86) International application number: PCT/JP2012/073514
(87) International publication number: WO 2013/039167

(56) References cited:
- EP-A1- 2 316 453
- WO-A1-2010/048716
- WO-A1-2012/106382
- JP-A- H02 215 719
- JP-A- H08 510 251
- JP-A- 2002 284 703
- JP-A- 2003 034 652
- JP-A- 2009 518 293
- JP-A- 2010 132 605
- US-A1- 2004 109 827
- US-A1- 2010 040 691

## Description

### TECHNICAL FIELD

Present invention relates to a pharmaceutical composition which can control the whole body exposure of a medicine, especially transmigration to skin of a medicine having a side effect of drug-induced photodermatosis. The present invention also relates to a respirable powder formulation which is easy to be handled pharmaceutically and makes it possible to retain the uniform drug content because of improvement in dispersibility.

### BACKGROUND ART

Inhalation therapy has been applied for treatment of lung and respiratory tract disease, diagnosis of disease, transrespiratory tract and transpulmonary whole body medication, prophylaxis of disease, transrespiratory tract immunity desensitization therapy, etc. as a medicinal use of transrespiratory. However, the adaptation-determining method of this therapy is fully examined at neither of the cases. Therefore, development of the corresponding respirable formulation is desired.
As features of a general respirable formulation, recognized are 1) quick expression of drug effects, 2) gradual reduction of side effects, 3) possibility of small dose administration, 4) avoidance of the first-pass effect, etc. When a target region is lung, the respirable formulation is equipped with further outstanding features by having a large surface area equal to small intestine. In applying the respirable formulation as a targeting therapy, it is necessary to consider a selection-criteria method of the respirable formulation from viewpoint of not only efficacy to disease but a generation method of medicine particles, arrival parts, and relevancy of the basic physical properties of medicine to them. Now, the respirable formulation is used for bronchodilator, mucosa solubilizer, antibiotic, antiallergic agent, steroid, vaccine, physiological saline, etc., and in the case of their clinical application, site of action of inhalant, mechanism of the action, composition, direction for use, etc. are considered to be of important factors.

Recent years, in treatment of bronchial asthma or chronic lung disease, a Dry Powder Inhaler (Dry Powder Inhaler, DPI) has come to attract attention. This form has advantage that, in addition of the feature as above-mentioned respirable formulation, medicine can be stored with stable form for a long period of time. In DPI, there is a close relationship between a particle diameter of medicine particles being respired by patient and a deposition to respiratory tract [Pharmacia (1997) Vol. 33, No.6, and 98-102], and the aerodynamics correlation is accepted in what kind of medicine particle diameter deposits inside trachea and lung. Specifically, it is generally known that the optimal sizes of medicine particles which can reach bronchus or lung are particles which have an aerodynamics diameter of about 1 to 6 µm [Int. J. Pharm. (1994) 101 and 1-13].
Preferably, particles of several µm or less reach alveolus, and since they are efficiently absorbed from lung mucosa and migrate into blood, the particle size becomes important. However, the more the particles get fine, the more the fluidity of powder gets worse and, as a result, decline of filling precision and handling property at the time of production gets worried. Then, in order to solve these problems in handling of the DPI formulation, the method mentioned below is well known which mixes micronized particles with coarse particles, such as lactose and erythritol, being used as carrier. According to this method, by making micronized particles adhere to the carrier surface via intermolecular interaction, cohesive force of micronized particles become weaker, and the particle diameter becomes large further as a whole, and thus, the fluidity of the formulation is improved. The other methods including granulation of a medicine and a surface treatment method are mentioned (Patent Document 1).

Here, pirfenidone (henceforth PFD) is the world's first anti-fibrosis agent for approval acquisition to be applied for idiopathic pulmonary fibrosis. The action mechanism is production modulation for various cytokines, such as inflammatory cytokine and anti-inflammation cytokine, and for growth factors which participate in fibrosis formation, and the anti-fibrosis effect is shown based on complex effects, such as fibroblast multiplication depressant action and collagen production depressant action. In comparison between this agent and prednisolone, while prednisolone showed only anti-inflammatory activity, this agent showed both anti-inflammatory activity and anti-fibrosis effect, then, consequently, it is expected that more effective therapeutic results than steroid can be brought about. Although it has been sold since 2008 in Japan, and is widely used for pulmonary fibrosis, many of patients who have taken this agent have showed a side effect of drug-induced photodermatosis, and their expression frequency results in about 60 percent. In order to avoid this problem, suitable dosage form which can easily show effect on lung local part has been desired. However, only oral formulation has been marketed till the present, and more preferable dosage form design aiming at stability and local administration has not been examined irrespective of the high demand. That is, development is desired strongly for new dosage forms which will reduce photodermatosis risk, a side effect of pirfenidone, and will bring about safer pulmonary fibrosis treatment. As DPI formulations in which micronized particles were made to adhere to the carrier surface, a formulation using lactose as a carrier (Patent Document 2), a cyclosporin formulation (Non-patent Document 1), a tranilast formulation (Patent Document 3, Non-patent Documents 2-3), etc. have been reported. However, in any references, the transmigration control to the skin and the photodermatosis risk fall of medicine by the above-mentioned DPI formulation are not described.

### Lists of prior arts:

[Patent Document 1] WO99/27911
[Patent Document 2] Japanese Patent 4125512
[Patent Document 3] Japanese Patent Publication 2011-93849 A

[Non-patent Document 1] Journal of Controlled Release (2009), 138(1), 16-23
[Non-patent Document 2] Journal of Pharmaceutical Sciences (2011), 100(2), 622-633
[Non-patent Document 3] European Journal of Pharmaceutics and Biopharmaceutics (2011), 77(1), 178-181

WO 2012/106382 discloses formulations of pirfenidone and pyridone analog compounds for aerosolization and the use of such formulations for aerosol administration of pirfenidone or pyridone analog compounds for the prevention or treatment of various fibrotic and inflammatory diseases.

US 2010/0040691 relates to compositions comprising methotrexate, preferably wherein the compositions are for administration by the inhaled or intranasal route.

US 2004/0109827 provides a powder formulation that can be obtained by mixing fine particles containing powdery medicament and excipient and having an average particle diameter of not more than 20 µm with a carrier having an aerodynamically acceptable particle diameter.

JP 2010-132605 relates to a pharmaceutical composition containing a polymer compound and a drug compound such as cyclosporine in a state of fine particles or adsorbed to the polymer compound.

JP 2003-034652 relates to a powder preparation obtained by mixing fine particles containing powdery drug and excipient, having an average particle diameter of 20 µm or less, with a carrier.

JP 2002-284703 relates to a powder pharmaceutical preparation obtained by mixing fine particles containing a drug with a sugar alcohol as a carrier having an aerodynamically acceptable particle diameter.

JP HO2 215719 relates to 5-methyl-1-phenyl-2-(IH)-pyridone as a antiphlogistic agent containing antipyretic and analgesic action as an antifibrosing action ingredient.

JP HO8 510251 relates to N-substituted 2-(IH) pyridones and/or N-substituted 3-(IH) pyridones for use in the therapy of fibrotic disease.

WO 2010/048716 relates to a method of treating, preventing or reducing fibroproliferative disorders by administering a composition comprising a cytokine modifier such as pirfenidone or tranilast and an antioxidant which is a precursor or glutathione.

JP 2009-518293 relates to methods for reducing adverse events in patients receiving pirfenidone.

### SUMMARY OF INVENTION

### PROBLEMS TO BE SOLVED BY THE INVENTION

Medicines migrate to the whole body generally via blood by oral administration, then accordingly, they migrate also to the skin to some extent, and this is thought to causes a side effect. Accordingly, a subject of the invention is to provide a formulation which can control the whole body exposure of a medicine having a side effect of drug-induced photodermatosis, especially transmigration of the medicine to the skin. Still more preferably is a provision of a respirable formulation wherein the medicine, especially pirfenidone, shows a sufficient efficacy and outstanding inhalation characteristics.

### MEANS FOR SOLVING THE PROBLEMS

Inventors of the present invention repeated researches extensively to solve the above-mentioned problems, and as a result, they came to complete the present invention. That is, pirfenidone, which is a medicine having a side effect of drug-induced photodermatosis, was ground by a grinder such as a jet mill in the coexistence of excipients to afford micronized particles having a diameter which can reach lung aerodynamically, and subsequently, the micronized particles obtained were mixed well with carriers which have a good conformity with the obtained-micronized particles and have a diameter which can reach systema respiratorium aerodynamically, then a formulation with very high content-uniformity was successfully obtained to complete the present invention. When an experimental lung inflammation model rat was medicated with this formulation in respiratory tract, while a control group showed very high lung disorder property and neutrophilic-leukocyte inflammation, a group administered with pirfenidone in a respirable formulation was able to be controlled powerfully against these conditions. When pirfenidone of dose (30mg/kg) which does not show anti-inflammatory activity was administered orally to rat, photodermatosis was not caused, but transmigration to the skin was observed promptly. On the other hand, when pharmacologically effective dose, for example, of 0.1mg/kg or more of the respirable powder formulation of pirfenidone was administered to the subject in respiratory tract, as compared with oral administration, significant suppression of the skin extraction rate was observed. From these data, it can be said that the respirable powder formulation of the present invention decreases dose remarkably by delivering a medicine directly to the pharmacodynamic target tissue, and moreover, in connection with it, the formulation shows such a prominent effect that a drug-induced photodermatosis risk which is a critical side effect may be reduced. The present invention is defined by the claims.

### EFFECTS OF THE INVENTION

According to the pharmaceutical composition of the present invention, it is possible to aerosolize easily a medicine powder, comprising pirfenidone, which has a side effect of drug-induced photodermatosis, and by delivering the medicine very specifically to lung, treatment of inflammatory lung disease, pulmonary fibrosis, etc. is remarkably enabled by low dose compared with an oral administration, and simultaneously, by preventing a skin transmigration of the medicine, it is possible to reduce the photodermatosis risk which is a main side effect of the medicine. And, the pharmaceutical composition of the present invention can be produced more preferably as a uniform-content formulation.

### BRIEF DESCRIPTION OF DRAWINGS

[Figure 1] Figure 1 shows a particle size distribution at the time when the formulation 1 was aerosolized.
[Figure 2] Figure 2 shows a SEM image at the time when carriers and micronized particles (excipient and pirfenidone particles) formed complexes.
[Figure 3] Figure 3 shows the result of photoreactivity test of each compound. In Figure 3 (A), the symbols represent; □/■:pirfenidone, ∇/▼:8-methoxy psoralen (MOP), and Δ/▲:sulisobenzone (□ ∇ Δ are singlet oxygen and ■ ▼ ▲ are superoxide). In Figure 3 (B), symbols represent; □:pirfenidone solution, ◊:pirfenidone powder, and ●: respirable powder formulation of pirfenidone.
[Figure 4] Figure 4 shows the amount of pirfenidone in each stage in the body of cascade impactor in the case of use or non-use of the carrier.
[Figure 5] Figure 5 shows a counting result of inflammatory cells in a broncho alveolar lavage fluid (BALF) of an asthma and chronic obstructive pulmonary disease model rat being administered with the formulation 1 by inhalation.
[Figure 6] Figure 6 shows results of biomarker measurement result of inflammatory cells in broncho alveolar lavage fluid (BALF) of the asthma and chronic obstructive pulmonary disease model rat being administered with the formulation 1 by inhalation.
[Figure 7] Figure 7 shows the amount of transmigration of pirfenidone to each tissue at the time of carrying out single-dose administration of the oral and respirable formulations. Symbols represent; Δ: oral pirfenidone 160mg/kg, ∇ :oral pirfenidone 30mglkg, and ● : 1mg/kg of the respirable powder formulation of pirfenidone.
[Figure 8] Figure 8 shows a concentration change of pirfenidone in skin at the time of administering oral and respirable formulations repeatedly. Symbols represent; Δ:oral pirfenidone 160mg/kg, ∇ :oral pirfenidone 30mg/kg, and ●:1mg/kg of the respirable powder formulation of pirfenidone.

### EMBODIMENTS TO CARRY OUT THE INVENTION

Hereinafter, the present invention is explained in detail.
[1] Medicine having a side effect of drug-induced photodermatosis Especially as medicines having a side effect of drug-induced photodermatosis, the medicines described in Table 2 of Current Drug Safety, (2009), vol 4, pp 123-126 are mentioned. For example, they are antibiotic, anticancer agent, antiepileptic drug, antidepressant, antifungal, antihistamine, antimalaric, gout drug, psychotropic drug, cardiovascular treating agent, diuretic, antilipemic, non-steroid anti-inflammatory agent, phototherapy agent, retinoid, pulmonary fibrosis treating agent, etc. Ciprofloxacin, enoxacin, lemefloxacin, sulfanilamide, sulfamethoxazole, tetracycline, etc. are mentioned as antibiotic. Fluorouracil, methotrexate, etc. are mentioned as the anticancer agent. Carbamazepine, phenobarbital, etc. are mentioned as the antiepileptic drug. Amitriptyline, amoxapine, etc. are mentioned as the antidepressant. Flucytosine, itraconazole, etc. are mentioned as the antifungal. Bromopheniramine, diphenhydramine, etc. are mentioned as the antihistamine. Chloroquine, kinin, etc. are mentioned as the antimalaric. Benzbromarone etc. are mentioned as the gout suppressant. Chlorpromazine, haloperidol, etc. are mentioned as the psychotropic drug. Captopril, clofibrate, etc. are mentioned as the cardiovascular treating agent. Furosemide, acetazolamide, etc. are mentioned as the diuretic. Glibenclamide, tolbutamide, etc. are mentioned as the antilipemic. Indomethacin, ibuprofen, etc. are mentioned as the non-steroid anti-inflammatory agent. 8-MOP (xanthotoxin), foscan, photofrin, etc. are mentioned as the phototherapy agent. Acitretin, etretinate, isotretinoin, etc. are mentioned as the retinoid. Pirfenidone is mentioned, as a treatment agent for pulmonary fibrosis. In the present invention, pirfenidone is used. As pirfenidone being used in the present invention, the method of preparing the same is not limited in particular, and what is used as drugs is included, such as crystal, amorphous, salt, hydrate, and solvate thereof.
[2] Excipient
   The excipient used herein is the one which is generally used for the purpose of gain in weight, dilution, filling, supporting of form, etc. of solid preparations, such as powders and a tablet. The excipient is effective in order to improve the solubility of a medicine, and/or to reduce the self-condensation ability of a medicine. Accordingly, although a water-soluble excipient is preferred, the excipient which absorbs moisture remarkably is not preferred for the property of this formulation. The excipient which is biologically inactive and is expected to be metabolized to some extent may be used. Water-soluble polymers can also be used, and there is no limitation for them as far as they are allowable as a medicine. Specific examples of such excipients that may be used include saccharides such as lactose, glucose, saccharose, trehalose, sucrose; sugar alcohols such as erythritol, mannitol, sorbitol; starches; macromolecular polymers such as crystalline cellulose, methyl cellulose, hydroxypropyl cellulose, hydroxypropyl methylcellulose, carmellose sodium, pullulan, dextrin, gum arabic, agar, gelatin, tragacanth, sodium alginate, polyvinylpyrrolidone, polyvinyl alcohols; stearic acid; fatty acids and salts thereof; waxes; calcium sulfate; calcium carbonate; talc; titanium oxide; and light anhydrous silicic acid. A combination of one or more sorts selected from these may be used. Preferable excipients in the present invention are one or more selected from the group consisting of sugars, sugar-alcohols, macromolecular polymers and calcium carbonates, and lactose or saccharose is preferable as sugars, erythritol, sorbitol, or mannitol is preferable as sugar-alcohols, and carmellose calcium, pullulan, polyvinylpyrrolidone, or methylcellulose is preferable as macromolecular polymers. Especially preferable excipient is lactose or erythritol.
[3] Carrier
   In the present invention, a carrier is used for preventing the medicine to condense before the administration of the powder formulation, and for improving absorption efficiency as the transpulmonary respirable formulation at the time of administration, by forming a complex between the medicine and the excipient (mentioned afterward). Especially, when an inhalation operation using an inhaler is carried out for the purpose of application in bronchus or lung, the inhaler is used for efficiently separating the excipient from the medicine after inhalation and, as a result, for improving absorption efficiency of the medicine. When the carrier is used for DPI formulation design, it is desirable to release the medicine definitely from the capsule or device, and to separate the medicine from the carrier surface with high probability. It is necessary to perform the formulation design in considering enough these points. In the case of using carriers, fluidity of the formulation, prevention of the medicine aggregation, and the propriety of dose increase and decrease, etc. become important. From these viewpoints, carriers in the present invention are preferably powdered. As selection criteria of carriers, easiness and workability for handling are required, not to mention toxicity or physicochemical stability. In order to clear these problems, lactose, the stability of which is also established conventionally, and is neutral, has little reactivity, and also has sweet taste a little, is useful in many points, and its usefulness is confirmed as the carrier for DPI [Int. J. Pharm. (1998) 172, 179-188]. As carriers which can be used in the present invention, sugars such as glucose, fructose, saccharose, maltose and dextran besides lactose, sugar-alcohols such as erythritol, sorbitol, and mannitol, common excipients such as calcium sulfate, calcium carbonate, talc, and titanium oxide can be mentioned also, and there is no limitation in particular. Preferable carrier is sugars or sugar-alcohols, and more preferable carrier is lactose or erythritol and, lactose is especially preferable.
   When the pharmaceutical composition of the present invention is a form being administered to the patient using an inhaler, the carrier is the one which has a particle diameter permitted aerodynamically. Specifically, a range of the mean particle diameter of the carrier is 10∼200 µm.
   When you want to make the carrier act only as a carrier from the point of dosage form design, it is known that enlarging of the particle diameter is enough for it. However, simultaneously, if the particle diameter is enlarged, it is also a well-known fact that the carrier retains at throat or the oral cavity. Accordingly, when it is more desirable to prevent the carrier itself from reaching even lung in spite of its biological inactivity, it is satisfactory if the mean particle diameter shall be at least 10 µm or more. When further best conditions are required, material selection after considering the conformity etc. of a main agent and a mixed excipient is desired. However, unless big problems are observed in particular, it is preferable to select a carrier of the same quality of material as that of the excipient.
[4] Mixing and grinding process of the medicine having a side effect of drug-induced photodermatosis and the excipient Manufacture of the powder formulation for inhalation administration may contain a mixing and grinding process of the medicine having a side effect of drug-induced photodermatosis and the excipient. Trituration is performed, for example, at the same time as the excipient and the medicine having a side effect of drug-induced photodermatosis are mixed using an aerodynamic grinder. The method is not limited for producing the powder formulation of the present invention, but a suitable method which a person skilled in the art usually uses can be used. It can be suitably determined by the kinds of medicine and excipient, the size of final particles, etc. whether which method is used or not. A crystallized state of the compound and the formulation characteristics, such as adhesion and dispersing ability, are correlated in many cases, and, consequently the latter processing method should be selected desirably in this step. However, in the case where pirfenidone is used as the medicine expressing a side effect of drug-induced photodermatosis, and erythritol is used as the excipient, because of the very high crystal orientation of pirfenidone, a good trituration mixture can be obtained even if any step is selected.
   Particles which comprise the obtained medicine and excipient according to the above-mentioned step are called micronized particles in this specification.
   Although a general drying-grinding process can be used for trituration of the medicine and the excipient, it is preferred to use especially an aerodynamic grinder. Specifically, devices which grind small quantity efficiently in a laboratory, such as a mortar and a ball mill, are used frequently as a common drying-grinding machine. As a ball mill, a rolling ball mill, a centrifugal ball mill, a vibration ball mill, and a planetary ball mill are known, and these can perform trituration by principles, such as grinding, rotation, vibration, and impact. There are many devices, as industrial use, aiming at efficiently grinding a lot of materials, such as a medium churning, type mill, a high velocity revolution trituration and impact mill, and a jet mill. There are a disc mill and a roller mill as a high velocity revolution trituration mill, and as a high velocity revolution impact mill, there are devices such as a cutter mill (knife mill), a hammer mill (atomizer), a pin mill, a screen mill, etc., which perform trituration also according to rotation impact in addition to shearing. As a jet mill, many mainly perform trituration with impact. As for the kind, there are the most orthodox particle-particle collision type, a particle-collision plate collision type, and a nozzle sucking type (blow off). In particular, it is preferred that the trituration is performed by the jet mill.
   As for a weight ratio of the medicine having a side effect of drug-induced photodermatosis and the excipient in the formulation of the present invention, a range of 1:5000∼10:1 is preferable. If the medicine increases more than this range, trouble may result in the content uniformity, and if the excipient increases more than this range, for a certain kind of medicine, there is a danger of elimination of the pharmacological activity. The weight ratio of the medicine having a side effect of drug-induced photodermatosis and the excipient is more preferably 1:100∼5:1, and still more preferably, 1:10∼2:1, and most preferably, 3:2.
   By grinding process, the medicine having a side effect of drug-induced photodermatosis is mixed with the excipient by homogeneity, and it is ground so that the mean particle diameter of the micronized particles becomes 1∼9 µm, preferably 3∼8 µm. The diameter within these ranges makes it possible for the micronized particles to reach the part of objects, such as bronchus and lung.
[5] Mixing step of carriers and micronized particles Subsequently, the micronized particles obtained in the above-mentioned mixing/trituration step are mixed with carriers, and till the time of administration, and let stable complexes to be formed. In the specification of the present application, the complex represents a molecular aggregate formed by condensation of a medicine with an excipient and a carrier via self-condensation capability caused by a molecular interaction of the medicines. Mixing of carriers and micronized particles can be performed by using a well-known mixer generally. There are mainly a batch system and a continuous system in the mixer, and further in the batch system, there are two sorts of a rotated type and a fixed mount type. There are a horizontal drum mixer, a V shaped rotary mixer, a double cone type mixer, and a cubic type mixer in the rotated type, and there are a screw type (perpendicularity, level) mixer, a revolution screw type mixer, and a ribbon type (perpendicularity, level) mixer in the fixed mount type. The continuous system is also divided into two sorts, a rotated type and a fixed mount type. As for the rotated type, a horizontal drum mixer and a level cone type mixer are known, and a screw type (perpendicularity, level) mixer, a ribbon type (perpendicularity, level) mixer, and a rotation disk type mixer are known for the fixed mount type. In addition, the mixing method using aerodynamic grinders, such as a medium churning type mill, a high velocity revolution grinding and an impact mill, and a jet mill, is possible. It is possible to make a uniform mixed-preparation by using and agitating a container which consists of product made of nylon, polyethylene, or the material having property similar to them.
   It is preferred to make the weight ratio of micronized particles and carriers into the range of 1:100∼10:1. If the micronized particles increase more than this range, trouble may result in the content uniformity, and if the carriers increase more than this range, for a certain kind of medicine, elimination - of the pharmacological activity is worried about. Weight ratios of micronized particles and carriers are more preferably 1:50 - 1:1, still more preferably 1:20 - 1:5 and most preferably 1:10.
   A ratio of mean particle diameters of micronized particles and carriers is preferably in the range of 1:1 - 1:50, and more preferably 1:5 ∼ 1:20.
[6] Inhaler
   When the complex obtained in the above-mentioned step is administered to a patient as the powder formulation for inhalation administration, the subject can be medicated by per-mucosal administration such as transpulmonary administration, nasal administration, etc. When the route of administration is transpulmonary administration, specifically, the powder formulation can be prescribed for the patient by using any inhalers generally used in the art.
   As the inhaler, devices for inhalation transpulmonary, such as Spin haler, E-haler, Flow-Caps, Jet haler, Disk haler, Rotor haler, Inspirer ease, Inhalation eight, etc. and quantitative atomizers, etc., can be used, but it is not limited to these.

### Example 1

### (1) Preparation of micronized particles used for respirable powder formulation

After mixing a pirfenidone crystal (about 60 mg) with various excipients (about 40 mg), micronization was performed with a jet mill, and thus, micronized particles were prepared. As the excipient, erythritol (Nikken formation), lactose (DMV), carmellose calcium (Daicel Chemical Industries), pullulan (Hayashibara), polyvinyl pyrrolidone (BASF), methyl cellulose (Shin-Etsu Chemical), sorbitol (Kao), calcium carbonate (Kanto Kagaku) or white soft sugar (Mitsui Sugar) was used.
(Grinding conditions)

| | |
|---|---|
| Used instrument: | A-O-Jet Mill (Seishin Enterprise) |
| Feeding method: | Auto feeder |
| Supply air pressure: | 6.0 kg/cm²G |
| Grinding air pressure: | 6.5 kg/cm²G |
| Dust collecting method: | Outlet bug (polyethylene) |

The yield was as follows, respectively.

| | | |
|---|---|---|
| Micronized particles 1 (excipient: erythritol) | | 75.8% |
| Micronized particles 2 (excipient: lactose) | | 61.0% |
| Micronized particles 3 (excipient: carmellose sodium) | | 59.9% |
| Micronized particles 4 (excipient: pullulan) | | 74.6% |
| Micronized particles 5 (excipient: polyvinyl pyrrolidone) | | 68.5% |
| Micronized particles 6 (excipient: methyl cellulose) | | 71.3% |
| Micronized particles 7 (excipient: sorbitol) | | 88.3% |
| Micronized particles 8 (excipient: mannitol) | | 68.4% |
| Micronized particles 9 (excipient: calcium carbonate) | | 72.9% |
| Micronized particles 10 (excipient: white soft sugar) | | 60.8% |

### (2) Preparation of respirable powder formulation

The micronized particles obtained in (1) were put into a STAT-3S antistatic bag made from polyethylene (20x30 cm, Asanuma industrial Corporation Ltd.) with carriers, and sealed after being filled with air, and the content was mixed by shaking by hand for about 3 minutes, then the formulations 1-20 shown in Table 1 were obtained. Samples were taken from five places arbitrarily after mixing, the amount of the drug contained was measured by UPLC/ESI-MS, and the uniformity of the content was thus confirmed. At this time, erythritol (Nikken formation, mean particle diameter: 20∼30 µm) or lactose (DMV, mean particle diameter: 50∼60 µm) was used as carriers. The weight ratio of the micronized particles and the carriers was 1:10.

### Example 2

### Particle-size-distribution measurement of respirable powder formulation

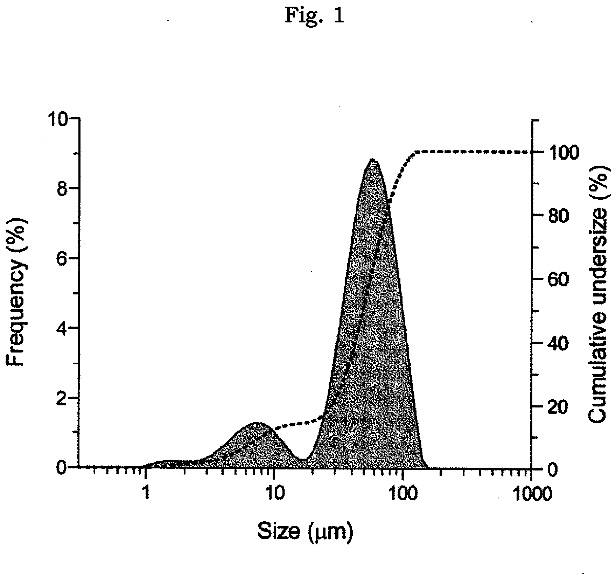

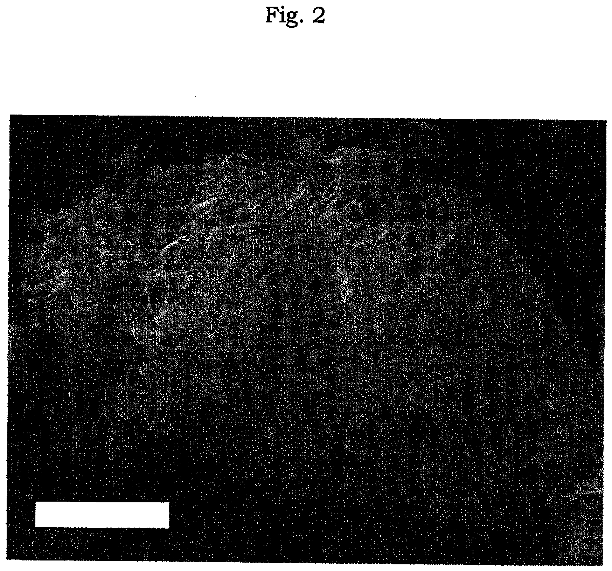

As a result of evaluating the mixture of micronized particles and carriers using a dry type laser diffraction device (LMS-300, Seishin Enterprise), aerosolization of any formulation was easily carried out under pressure of 0.2MPa. Figure 1 shows a particle size distribution of the mixture (formulation 1) of the micronized particles 1 and lactose carriers. Two main peaks are mainly shown, and the peak with a mean particle diameter of 7 µm originates in the micronized particles, and the peak with a mean particle diameter of 60 µm originates in the carriers. As for other formulations, the ranges of a mean particle diameter of the micronized particles were 3.0∼8.0 µm. It is considered that the carriers remain in respiratory tract at the time of inhalation, and the micronized particles may reach bronchus or lung at the time of inhalation. The mean particle diameters of the micronized particles in the formulations 1∼20 obtained by analysis are shown in Table 1. The SEM image which photographed the situation where the micronized particles (excipient and pirfenidone grains) were actually complexed with carriers (lactose) is shown (Figure 2). It can be observed that the pirfenidone grains, which were micronized by jet milling and turned into single spherical grains, adhered to lactose without any significant agglomeration.

**[Table 1]**

| | Excipient | Carrier | Meanparticle diameter of Micronized particles (µm) |
|---|---|---|---|
| Formulation 1 | erythritol | lactose | 7.0 |
| Formulation 2 | lactose | lactose | 5.6 |
| Formulation 3 | carmellose sodium | lactose | 7.1 |
| Formulation 4 | pullulan | lactose | 6.4 |
| Formulation 5 | polyvinylpyrrolidone | lactose | 6.5 |
| Formulation 6 | methyl cellulose | lactose | 7.3 |
| Formulation 7 | sorbitol | lactose | 3.7 |
| Formulation 8 | mannitol | lactose | 6.2 |
| Formulation 9 | calcium carbonate | lactose | 7.8 |
| Formulation 10 | saccharose | lactose | 4.6 |
| Formulation 11 | erythritol | erythritol | 5.3 |
| Formulation 12 | lactose | erythritol | 7.6 |
| Formulation 13 | carmellose sodium | erythritol | 3.8 |
| Formulation 14 | pullulan | erythritol | 5.6 |
| Formulation 15 | polyvinylpyrrolidone | erythritol | 4.1 |
| Formulation 16 | methyl cellulose | erythritol | 3.9 |
| Formulation 17 | sorbitol | erythritol | 7.4 |
| Formulation 18 | mannitol | erythritol | 4.8 |
| Formulation 19 | calcium carbonate | erythritol | 6.2 |
| Formulation 20 | saccharose | erythritol | 3.6 |

### Example 3

### Photoreactivity testing and photostability testing

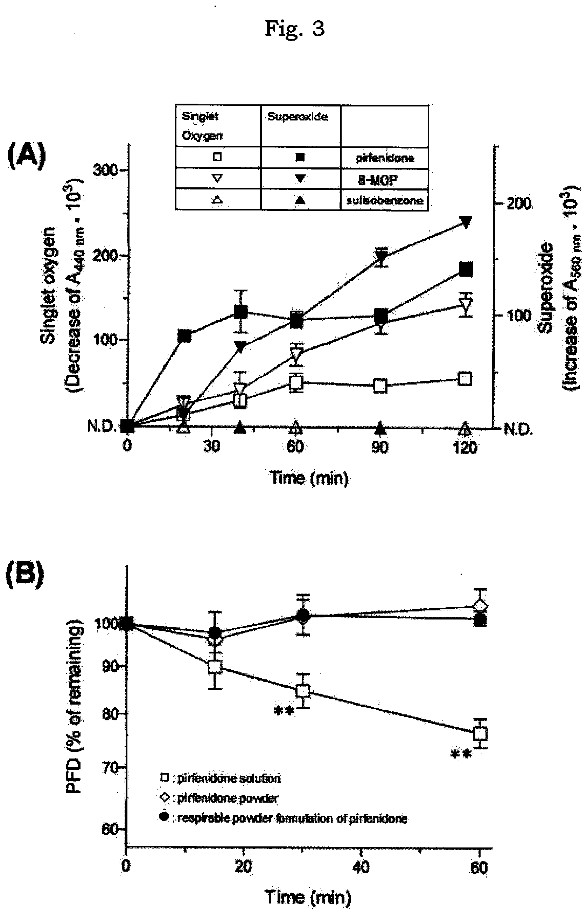

Figure 3 (A) shows the result of a reactive oxygen species (ROS) assay which was carried out to measure the photoreactivity of pirfenidone. This assay was established to monitor the generation of ROS, such as singlet oxygen and superoxide, from photoirradiated chemicals, and ROS generation would be indicative of the photochemical reactivity of tested chemicals. Sulisobenzone, a potent UV absorber, has no ability to generate ROS when exposed to simulated sunlight (250 W/m²); therefore, sulisobenzone can be identified to be less photoreactive. In contrast, 8-methoxypsoralen (MOP) is known as a phototoxic and photoreactive chemical. Although 8-MOP slightly exceeded pirfenidone in ROS generation, both chemicals were identified to be photoreactive, as shown in Figure 3 (A). When such photoreactivity and photoirritability are taken into consideration, pirfenidone can be said to raise concerns about photostability. Here, in respirable formulation of pirfenidone, both liquid and dry powder formulations can be considered in theory. Then, to clarify the photochemical properties in more detail, photostability testing was carried out. First, none of the pirfenidone samples tested showed any degradation in chromatographic analysis when they were stored at 25°C for 1 h under light protection. Exposure of PFD solution to simulated sunlight (250 W/m²) resulted in the degradation of pirfenidone, and it was likely to follow first-order kinetics with an apparent first-order degradation rate of 0.31±0.03 h⁻¹ as shown in Figure 3 (B). In contrast, no significant degradation was observed in pirfenidone powder under the same irradiation conditions. In general, photosensitive chemicals in a solution state are far more prone to photodegradation than solid samples due to the high permeability of light and increased mobility of photochemically excited molecules and reactive species. From this, taken together with the result shown in Figure 3 (B), dry powder formulation, rather than liquid formulation, is preferable as a respirable formulation of pirfenidone.

### Example 4

### Assessment of the respirable powder formulation (formulation 1) by cascade impactor

In order to conduct investigation on the aerodynamic particle size of fine powders, an examination was carried out using a cascade impactor which is an artificial respiratory tract and a lung model. A body of the impactor is composed of piles in eight stages and a final filter, combined with a velocity indicator and a suction pump. The fundamental method, which is a procedure described in "Multistage Cascade Impactor Apparatus" of USP 2000 "Physical Tests and Determinations/Aerosols" was applied. The specified method is as follows.

### (Method)

Apparatus: Andersen sampler (AN-200, product of Shibata chemicals)
Pump flow rate: 28.3 L/min
Device used: Jet-Haler (made by UNISIA JECS)
Sample: (i) formulation 1 respirable powder formulation
(Mixing ratio; micronized particles 1 ground by jet mill: carriers = 1:10)
(ii) Micronized particles 1
(ground by jet mill, but not mixed with carriers)
A Japanese Pharmacopoeia No. 2 capsule was filled up with samples (i) and (ii) in proper quantity, respectively, and was installed in the device.
Drug determination method: (UPLC-MS analysis conditions)

| | | |
|---|---|---|
| Column used: | Acuity UPLC BEH C 18 Column (Waters) | |
| Detector: | SQ Detector (Waters) | |
| Pump: | Binary Solvent Manager (Waters) | |
| Flow rate of mobile phase: | | 0.25 mL/min |
| Mobile phase: | A:100% acetonitrile, B: 5 mM ammonium acetate | |
| | 0∼1 Min.: | A 20% |
| | 1∼3 Min.: | A 20-95% |
| | 3∼4 Min.: | A 95% |
| Column temperature: 40 °C | | |

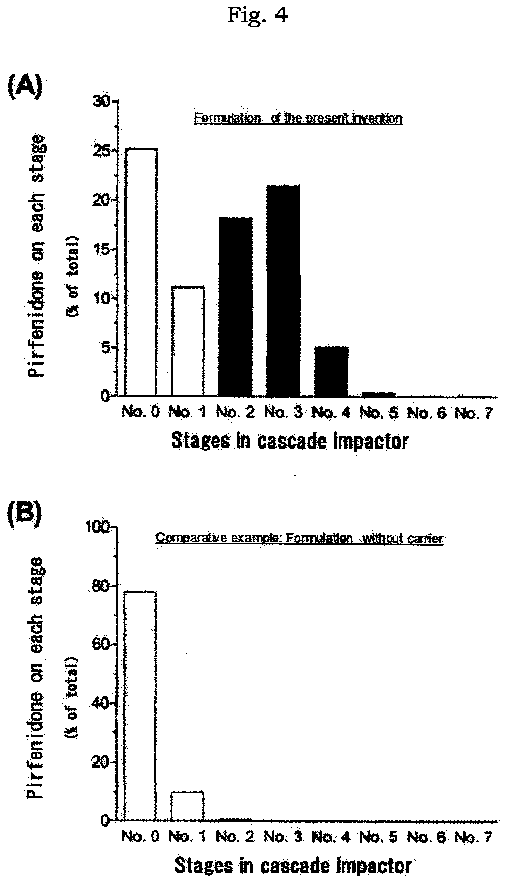

(Results) The amount of pirfenidone in each stage in the body of cascade impactor is shown in Figure 4. From assessment of aerodynamic particle size by cascade impactor, as shown in the graph of Figure 4 (A), the respirable powder formulation (Formulation 1) of sample (i) was found to be mainly distributed in the stage 0 and the stages 2∼4. The particles distributed in the stage 0 are estimated to be pirfenidone contained in the undissociated complexes of micronized particles and carriers. The dissociated micronized particles were found to be mainly distributed in the stages 2∼4. The amount of percents for particles distributed in the stages 2∼7 is defined by RF value specified as "a rate with which micronized particles arrive at a target site, bronchial tubes or lung." The RF value in this Example exceeds 45%. Accordingly, it is thought that the respirable formulation of the present invention, which is a complex of micronized particles and carriers, remains in respiratory tract, and only micronized particles dissociated from the complex fully reach the target site, bronchial tubes or lung. As to the release from a capsule, high fluidity and dispersibility of the formulation were also shown, since 98.6% of the formulation was confirmed to be emitted from the capsule.

On the other hand, when the sample (ii), the micronized particles 1 using no carrier, was analyzed, about 99% remained in the stages 0 and 1, and the RF value was less than 1% as shown in the graph of Figure 4 (B). It is confirmed that sufficient dispersibility cannot be obtained without a carrier, and the fall of the inhalation properties was caused by forming agglomeration of the micronized particles.

Accordingly, in order to make the micronized particles reach the target site (bronchial tubes or lung), the powder respirable formulation using a carrier is preferred.

### Example 5

### (1) Preparation of a model animal sensitized with albumen origin ovalbumin (OVA) and medication of the respirable powder formulation (formulation 1) in airway

Using an OVA-sensitized animal model which is a typical asthma and chronic obstructive pulmonary disease model, a medicinal effect of the respirable powder formulation of the formulation 1 was assessed. This model causes local inflammation in the respiratory organ by prescribing the OVA respirable powder formulation in airway of the animal sensitized by the OVA acting as an antigen which causes neutrophilic leukocyte inflammation and eosinophilic leukocytosis in lung. The illustrative procedure of the model preparation and the medication in airway of the respirable powder formulation of the formulation 1 are shown below.

### (Procedure)

Animal: Sprague-Dawley rat (8-11 weeks of age)
Reagents: Albumen origin ovalbumin (SIGMA) and aluminum hydroxide gel (SIGMA)
Medication instrument in airway: DP-4 (Ina Research, Inc.)
Animals were sensitized by intraperitoneal injection of the OVA solution (OVA: 0.33 mg/kg with 16.6 mg of alum) on days 0, 7, and 14. They were received intratracheal administration of the OVA respirable powder formulation (100 µg as OVA amount) at 24 h after the last OVA sensitization. The intratracheal administration was performed by sending compressed air through inserted DP-4 in the airway after anesthetized with sodium pentobarbital.

To the control group, a respirable powder formulation produced by using lactose was used.

Pre-medication of the formulation 1 (1 mg/kg) was performed 1 hour before medication of the OVA respirable powder formulation.

**[Table 2]**

| | Pre-medication | 24 hrs. after final sensitization |
|---|---|---|
| OVA group | lactose- DPI | OVA-DPI |
| control group | lactose- DPI | lactose-DPI |
| Formulation 1 group | Formulation 1 | OVA-DPI |

### (2) Bronchoalveolar lavage fluid (BALF) collection, and total cell numbers in BALF

BALF is said to be useful for diagnosis of respiratory disease. In this Example, inflammation and tissue disorders were assessed by counting the total cells in BALF. At 24 h after the OVA challenge, BALF was collected by washing the airways with 5 mL of PBS by inserting cannula into the airway after brood removal from ventral aorta under anesthesia by Nembutal. The BALF collected was pooled and centrifuged for 5 min, the supernatant was then removed, and cells were re-suspended with 1 mL of PBS. The total number of cells in BALF was counted using a manual hemocytometer under microscope.

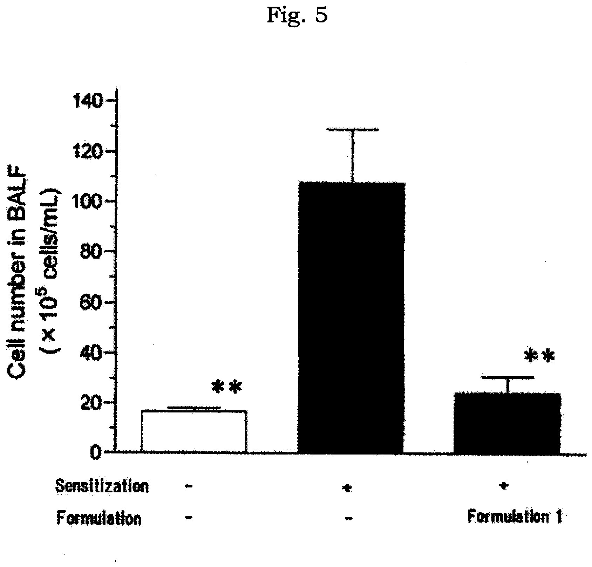

Figure 5 shows the result of measurement of the inflammatory cell infiltration inhibiting activities by the formulation 1 (1mg/kg) in an experimental asthma and chronic obstructive pulmonary disease model animal. The ordinate shows total cell numbers in BALF. The total cell numbers mainly consist of monocytes and neutrophils.

In the OVA group at 24 hours after the last sensitization, the total cell numbers increased by about 6.5-fold compared to that of the control group. On the other hand, in the formulation 1 group, the total cell numbers in BALF decreased by about 90% as compared to those of the control group.

Said inflammation decreased in a dose-dependent manner by pretreatment with the formulation 1 (0.1∼3.33 mg/kg), and the total cell numbers in BALF at the time of treatment by 3.33 mg/kg was almost equivalent to the case of treatment by 1mg/kg.

These data were indicative of the therapeutic potential of the formulation 1 against inflammation in the lung local part observed in pulmonary fibrosis, asthma, etc.

### (3) Measurement of lung inflammation injury biomarkers in BALF

In order to examine the pharmacologic effect of the formulation 1 in detail, various biomarkers in BALF were measured. Lactate dehydrogenase (LDH) was chosen as a biomarker of lung disorder, and myeloperoxidase (MPO) was chosen as a biomarker of neutrophilic leukocyte inflammation, respectively. In inflammation and fibrosis of the airway, MPO secreted from neutrophilic leukocyte/macrophage works as a pro-inflammatory mediator. Accordingly, the enzyme activity of MPO functions as a biomarker of neutrophilic leukocyte.

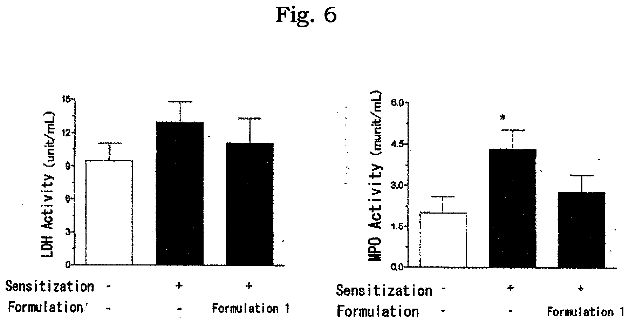

Measurement results of LDH activity and MPO activity are shown in Figure 6.

As compared to the control group, both of the LDH activity and the MPO activity in the OVA group at 24 hours after the last sensitization increased. On the other hand, in the formulation 1 group, reduction of each biomarker was observed as compared to the OVA group at 24 hours after the last sensitization. Specifically, an increase rate in which each biomarker increases from the control group by OVA sensitization was attenuated by medication of the formulation 1 to about 67% in MPO activity and to about 52% in LDH activity, respectively. Thus, the formulation 1 is thought to be efficacious for suppression of neutrophilic inflammation and imbalance of the enzyme system accompanying with it.

These observations were in agreement with the inhibitory effects on the recruitment of inflammatory cells in BALF and thus, the present data were also indicative of the topical therapeutic potential of the formulation 1 respirable powder formulation for the treatment of pulmonary inflammatory and fibrotic diseases.

### Comparative example 1

### Examination of phototoxic reaction in oral administration of pirfenidone

Hair of rats were carefully shaved by hair clipper, and pirfenidone was administered orally to the rats (160 mg/kg or 30 mg/kg), and the rats were irradiated with a black light. Colors of the skin before and after the light irradiation were evaluated with a color difference meter. The results are shown in Table 3. Change of skin color was intentionally observed in the group medicated with 160 mg/kg of pirfenidone compared to the control group, whereas significant change was not observed in the group medicated with 30 mg/kg of pirfenidone.

**[Table 3]**

| UV | | | evaluation of color change | | | |
|---|---|---|---|---|---|---|
| | | | L* | a* | b* | 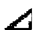 E |
| control | - | initial value after treatment | 64.91 | 1.26 | 7.19 | 1.13 ± 0.34 |
| | | | 65.98 | 1.03 | 7.07 | |
| | + | initial value after treatment | 71.22 | 1.30 | 2.29 | 226 ± 0.19 |
| | | | 69.23 | 1.92 | 3.03 | |
| pirfe nidone (160mg/kg) | - | initial value after treatment | 70.59 | 0.09 | 3.67 | 1.69 ± 0.24 |
| | | | 71.04 | -1.04 | 4.88 | |
| | + | initial value after treatment | 71.05 | 0.89 | 5.67 | 3.97 ± 0.59 |
| | | | 72.35 | 2.06 | 9.17 | |
| pirfenidone (30mg/kg) | - | initial value after treatment | 70.16 | 1.19 | -0.18 | 1.47 ± 0.27 |
| | | | 71.56 | 1.54 | -0.36 | |
| | + | initial value after treatment | 72.90 | 1.27 | 4.14 | 2.40 ± 0.20 |
| | | | 72.26 | 2.81 | 5.72 | |

### Example 6

### Pharmacokinetics (1) of pirfenidone at the time of airway administration of the formulation 1

The respirable powder formulation of pirfenidone resulted in marked decrease in the amount of necessary dose as compared to the oral administration. However, the possibility of risk reduction of photodermatosis is not clear. In general, since drug-induced photodermatosis appear in the skin and eyes, the specific distribution of drug molecules in the skin and/or eyes could be a key consideration for evaluating the photodermatosis risk. Then, in order to verify the photosafety of the formulation 1, a pharmacokinetic study was undertaken after intratracheal administration of the formulation 1 at a pharmacologically effective dose (1 mg/kg). Additionally, since in the oral administration, both pharmaceutically effective and phototoxic dose (160 mg/kg) and pharmaceutically non-effective and non-phototoxic dose (30 mg/kg) are known, pharmacokinetic parameters were obtained after oral administration at both doses.

Concentration-time curves of pirfenidone in the plasma, skin, lung, and eyes were obtained by UPLC/ESI-MS analysis after intratracheal and oral administrations. Relevant pharmacokinetic parameters of pirfenidone, including *C*ₘₐₓ, *t*_{1/2}, AUC_{0→∞}, and MRT, were summarized in Table 4. After oral administration of pirfenidone, plasma and lung concentrations of pirfenidone immediately reached the *C*ₘₐₓ within 5 min and these concentrations decreased steadily with half lives of ca. 0.3-0.8 h. With respect to skin and eye depositions, reaching maximum levels at ca. 0.5 h after oral dosing, followed by an elimination phase with a half-life of ca. 0.7-1.1 h. Thus, the elimination of pirfenidone from the skin and eyes was found to be slower than that in plasma and pirfenidone may accumulate in these light-exposed areas (skin and eyes) upon chronic dosing, resulting in an increased photodermatosis risk. In contrast, after intratracheal administration of the formulation 1, each of plasma and tissue concentrations of pirfenidone immediately reached the *C*ₘₐₓ within 5 min, and then, these medicaments rapidly diminished below detectable levels within 1.5 h.

The intra airway administration of the formulation 1 (1 mg/kg) led to ca. 440-, 90-, and 30-fold reductions in *C*ₘₐₓ values for plasma, skin, and eyes, respectively, compared to the orally-taken pirfenidone at the photodermatosis expression dose (160 mg/kg). The AUC values in plasma, skin, and eyes decreased by ca. 1,800-, 370-, and 440-fold, respectively. From these studies, it was confirmed that the intratracheal administration of pirfenidone successfully resulted in marked decrease in systemic exposure, compared to the oral administration.

In addition, there were still ca. 63∼70-fold differences in AUC values for skin and ocular pirfenidone between the oral formulation (30 mg/kg) and the respirable formulation (1mg/kg) at non-photodermatosis level of doses. The differences of these pharmacodynamics coefficients show that use by inhalation of pirfenidone decreases notably the accumulation in skin and eyes and the systemic exposure of pirfenidone compared to the oral administration. Even if compared with dose which does not reveal photodermatosis by taking orally, blood level after inhalation of the formulation 1 shows notably low value. This suggests that it becomes possible for application of this pharmaceutical technology to raise effect in lung local part of this agent, and to reduce remarkably the risk of onset of the side effect, photodermatosis, further.

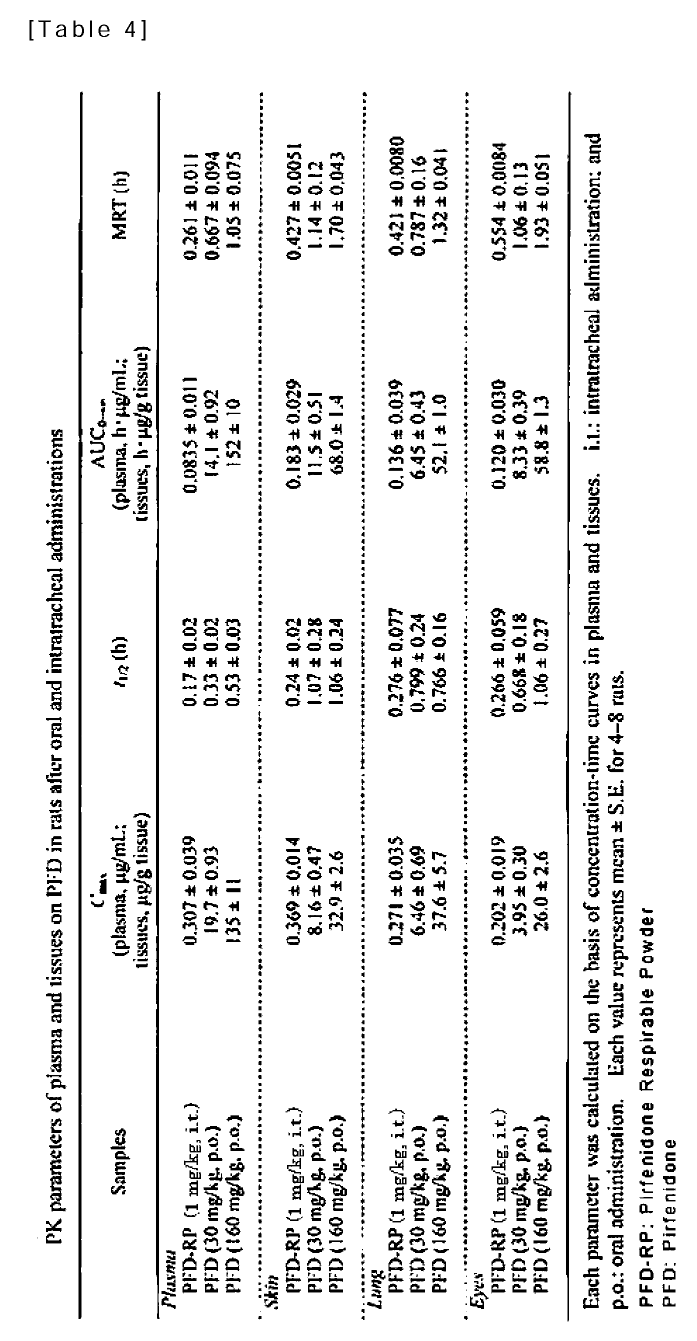

### Example 7

### Pharmacokinetics (2) of pirfenidone at the time of airway administration of the formulation 1

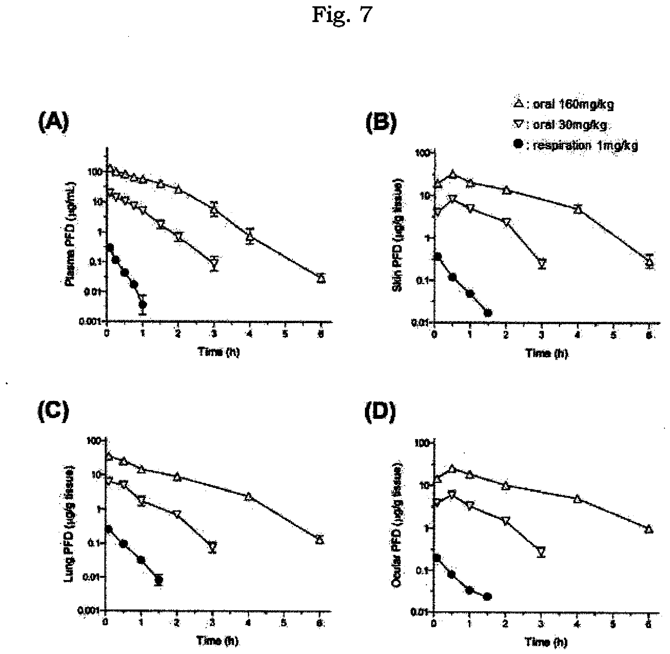

Similarly to Example 5, single-dose administrations of pirfenidone orally and the formulation 1 by inhalation were performed in rats, and the amount of transmigration of pirfenidone to each tissue was monitored by UPLC/ESI-MS. Results are shown in Figure 7. When compared in dose (160mg/kg) having an anti-inflammatory activity, while the amount of skin-transmigration of pirfenidone in 1 hour after administration was about 50 ng/g-tissue via inhalation (1 mg/kg), the amount via oral administration (160mg/kg) was about 20 µg/g-tissue. From this, while the amount of medication in inhalation is 1/160 in the case of oral administration, the amount of skin transmigration in inhalation decreases to 1/400 of oral formulation. Significant reduction of the amount of skin transmigration of pirfenidone like this suggests large mitigation of side effects by the present invention. Thus, the respirable formulation of the present invention has an excellent effect.

### Pirfenidone pharmacokinetics (3) at the time of airway administration of the formulation 1

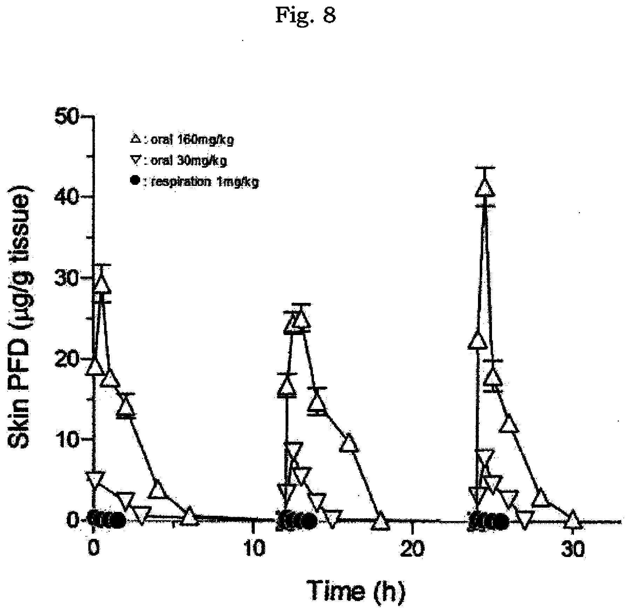

Results of having monitored the amount of skin transmigration of pirfenidone in repeated-dose administrations by oral and inhalation are shown in Figure 8. Although a temporary elevation of the pirfenidone concentration in the skin was observed by pirfenidone oral administration (30,160mg/kg) also in repeated-dose administrations in every 12 hours, the elevation disappeared in about 6 hours after administration. In repeated-dose administrations, pirfenidone did not show any accumulation trend. At the time of inhalation of the formulation 1, lower skin transitionality was observed more remarkably than the time of administration of oral dose (30mg/kg) which does not show photodermatosis, and similarly, no accumulation trend was shown. From this, the systemic side effect is considered to be avoidable even when the respirable formulation is used repeatedly.

### INDUSTRIAL APPLICABILITY

The present invention provides the powder formulation which reduces side effect risk of medicine having a side effect of a drug-induced photodermatosis and increases therapeutic effect, and the method for producing the same. Since the powder formulation of the present invention makes inhalation therapy possible by the ability of easy aerosolizability and thus increases pharmacological effect in lung local part, the reduction of dose becomes possible. The skin transmigration of the aforementioned medicine can be controlled with a lung specific delivery technology, and thus, photodermatosis, a side effect, can be controlled.

## Claims

1. A powder formulation comprising
(i) micronized particles with a mean particle diameter in the range of 1 to 9 µm comprising
(i-1) a drug showing a side effect of drug-induced photodermatosis which is pirfenidone and
(i-2) an excipient, and
(ii) a carrier with a mean particle diameter of 10∼200 µm.

2. The powder formulation according to Claim 1, wherein the drug showing a side effect of drug-induced photodermatosis further comprises a drug selected from the group consisting of antibiotics, anticancer drug, antiepileptic drug, antidepressant, antifungal, antihistamine, antimalarial, gout drug, psychotropic drug, cardiovascular remedy, diuretic, antilipemic, non-steroid anti-inflammatory agent, phototherapy agent, retinoid, and pulmonary fibrosis treating agent.

3. The powder formulation according to Claim 1 or 2, wherein the micronized particle and the carrier form a complex.

4. The powder formulation according to Claim 3, wherein a particle diameter of the micronized particle is smaller than a mean particle diameter of the carrier.

5. The powder formulation according to any one of Claims 1-4, wherein the excipient and/or the carrier are saccharides.

6. The powder formulation according to Claim 5, wherein saccharides are lactose.

7. The powder formulation according to any one of Claims 1-4, wherein the excipient and/or the carrier are sugar alcohols.

8. The powder formulation according to claim 7, wherein the sugar alcohols are erythritol.

9. The powder formulation according to any one of Claims 1-4, wherein the excipient is macromolecular polymers.

10. The powder formulation according to any one of Claims 1-4, wherein the excipient is erythritol and the carrier is lactose.

11. The powder formulation according to any one of Claims 1-10, wherein a ratio between the drug showing side effects of drug-induced photodermatosis and the excipient is in the range of 1:5000∼10:1 in weight ratio.

12. The powder formulation according to any one of Claims 1-11, wherein the ratio between the micronized particle and the carrier is in the range of 1:100∼10:1 in weight ratio.

13. The powder formulation according to any one of Claims 1-12, wherein the powder formulation is a transpulmonary inhalation formulation.

14. A process for producing the formulation according to any one of Claims 1-13, wherein the micronized particles with a mean particle diameter in the range of 1 to 9 µm comprising a drug showing side effects of drug-induced photodermatosis which is pirfenidone and an excipient are mixed with a carrier having a particle diameter of 10∼200 µm.

15. The process according to Claim 14, wherein the micronized particles are prepared by mixing the drug showing side effects of drug-induced photodermatosis and the excipient, and micronizing the mixture by a jet mill.

16. The process according to Claim 15, wherein the micronized particles and the carrier are mixed in a container made from nylon or polyethylene.

17. The process according to any one of Claims 14-16, wherein the micronized particle and the carrier form a complex.

18. An inhalation formulation obtained by the process according to any one of Claims 14-17.

## Patentansprüche

1. Pulverformulierung, umfassend
(i) mikronisierte Teilchen mit einem mittleren Teilchendurchmesser im Bereich von 1 bis 9 pm, umfassend
(i-1) ein Arzneimittel, das eine Nebenwirkung einer Arzneimittel-induzierten Photodermatose aufweist und welches Pirfenidon ist, und
(i-2) einen Hilfsstoff, und
(ii) einen Träger mit einem mittleren Teilchendurchmesser von 10∼200 µm.

2. Pulverformulierung gemäß Anspruch 1, wobei das Arzneimittel, das eine Nebenwirkung einer Arzneimittel-induzierten Photodermatose aufweist, ferner ein Arzneimittel, ausgewählt aus der Gruppe bestehend aus Antibiotika, Antikrebsmittel, antiepileptischem Arzneimittel, Antidepressivum, Antimykotikum, Antihistamin, anti-Malaria-Arzneimittel, Gicht-Arzneimittel, psychotropem Arzneimittel, kardiovaskulärem Mittel, Diuretikum, Lipidsenker, nichtsteroidalem entzündungshemmendem Arzneimittel, Phototherapiemittel, Retinoid und Lungenfibrose-Behandlungsmittel, umfasst.

3. Pulverformulierung gemäß Anspruch 1 oder 2, wobei das mikronisierte Teilchen und der Träger einen Komplex bilden.

4. Pulverformulierung gemäß Anspruch 3, wobei ein Teilchendurchmesser des mikronisierten Teilchens kleiner ist als ein mittlerer Teilchendurchmesser des Trägers.

5. Pulverformulierung gemäß mindestens einem der Ansprüche 1-4, wobei der Hilfsstoff und/oder der Träger Saccharide sind.

6. Pulverformulierung gemäß Anspruch 5, wobei die Saccharide Lactose sind.

7. Pulverformulierung gemäß mindestens einem der Ansprüche 1-4, wobei der Hilfsstoff und/oder der Träger Zuckeralkohole sind.

8. Pulverformulierung gemäß Anspruch 7, wobei die Zuckeralkohole Erythritol sind.

9. Pulverformulierung gemäß mindestens einem der Ansprüche 1-4, wobei der Hilfsstoff makromolekulare Polymere ist.

10. Pulverformulierung gemäß mindestens einem der Ansprüche 1-4, wobei der Hilfsstoff Erythritol ist und der Träger Lactose ist.

11. Pulverformulierung gemäß mindestens einem der Ansprüche 1-10, wobei ein Verhältnis zwischen dem Arzneimittel, das Nebenwirkungen einer Arzneimittel-induzierten Photodermatose aufweist, und dem Hilfsstoff im Bereich von 1:5000∼10:1 als Gewichtsverhältnis liegt.

12. Pulverformulierung gemäß mindestens einem der Ansprüche 1-11, wobei das Verhältnis zwischen dem mikronisierten Teilchen und dem Träger im Bereich von 1:100∼10:1 als Gewichtsverhältnis liegt.

13. Pulverformulierung gemäß mindestens einem der Ansprüche 1-12, wobei die Pulverformulierung eine transpulmonale Inhalationsformulierung ist.

14. Verfahren zur Herstellung der Formulierung gemäß mindestens einem der Ansprüche 1-13, bei dem die mikronisierten Teilchen mit einem mittleren Teilchendurchmesser im Bereich von 1 bis 9 pm, die ein Arzneimittel, das Nebenwirkungen einer Arzneimittel-induzierten Photodermatose aufweist und welches Pirfenidon ist, und einen Hilfsstoff umfassen, mit einem Träger mit einem Teilchendurchmesser von 10∼200 µm gemischt werden.

15. Verfahren gemäß Anspruch 14, bei dem die mikronisierten Teilchen durch Mischen des Arzneimittels, das Nebenwirkungen einer Arzneimittel-induzierten Photodermatose aufweist, und des Hilfsstoffes hergestellt werden und die Mischung durch eine Strahlmühle mikronisiert wird.

16. Verfahren gemäß Anspruch 15, bei dem die mikronisierten Teilchen und der Träger in einem Behälter, der aus Nylon oder Polyethylen hergestellt ist, gemischt werden.

17. Verfahren gemäß mindestens einem der Ansprüche 14-16, bei dem das mikronisierte Teilchen und der Träger einen Komplex bilden.

18. Inhalationsformulierung, erhältlich durch das Verfahren gemäß mindestens einem der Ansprüche 14-17.

## Revendications

1. Formulation de poudre comprenant
(i) des particules micronisées ayant un diamètre de particule moyen dans la plage de 1 à 9 µm comprenant
(i-1) un médicament présentant un effet secondaire de photodermatose induite par un médicament qui est la pirfénidone et
(i-2) un excipient, et
(ii) un vecteur ayant un diamètre de particule moyen de 10 ∼ 200 µm.

2. Formulation de poudre selon la revendication 1, où le médicament présentant un effet secondaire de photodermatose induite par un médicament comprend en outre un médicament choisi dans le groupe consistant en les antibiotiques, un médicament anticancéreux, un médicament antiépileptique, un antidépresseur, un antifongique, un antihistaminique, un antimalaria, un médicament contre la goutte, un médicament psychotrope, un remède cardiovasculaire, un diurétique, un antilipémique, un agent anti-inflammatoire non stéroïdien, un agent de photothérapie, un rétinoïde et un agent traitant la fibrose pulmonaire.

3. Formulation de poudre selon la revendication 1 ou 2, où la particule micronisée et le vecteur forment un complexe.

4. Formulation de poudre selon la revendication 3, où un diamètre de particule de la particule micronisée est plus petit qu'un diamètre de particule moyen du vecteur.

5. Formulation de poudre selon l'une quelconque des revendications 1-4, où l'excipient et/ou le vecteur sont des saccharides.

6. Formulation de poudre selon la revendication 5, où les saccharides sont le lactose.

7. Formulation de poudre selon l'une quelconque des revendications 1-4, où l'excipient et/ou le vecteur sont des sucres-alcools.

8. Formulation de poudre selon la revendication 7, où les sucres-alcools sont l'érythritol.

9. Formulation de poudre selon l'une quelconque des revendications 1-4, où l'excipient est des polymères macromoléculaires.

10. Formulation de poudre selon l'une quelconque des revendications 1-4, où l'excipient est l'érythritol et le vecteur est le lactose.

11. Formulation de poudre selon l'une quelconque des revendications 1-10, où un rapport entre le médicament présentant des effets secondaires de photodermatose induite par un médicament et l'excipient est dans la plage de 1 : 5000∼10 : 1 en rapport en poids.

12. Formulation de poudre selon l'une quelconque des revendications 1-11, où le rapport entre la particule micronisée et le vecteur est dans la plage de 1 : 100 ∼ 10 : 1 en rapport en poids.

13. Formulation de poudre selon l'une quelconque des revendications 1-12, où la formulation de poudre est une formulation pour l'inhalation transpulmonaire.

14. Procédé pour produire la formulation selon l'une quelconque des revendications 1-13, où les particules micronisées ayant un diamètre de particule moyen dans la plage de 1 à 9 µm comprenant un médicament présentant des effets secondaires de photodermatose induite par un médicament qui est la pirfénidone et un excipient sont mélangées avec un vecteur ayant un diamètre de particule de 10 ∼ 200 µm.

15. Procédé selon la revendication 14, où les particules micronisées sont préparées par mélange du médicament présentant des effets secondaires de photodermatose induite par un médicament et de l'excipient, et micronisation du mélange par un broyeur à jet.

16. Procédé selon la revendication 15, où les particules micronisées et le vecteur sont mélangés dans un récipient fait de nylon ou de polyéthylène.

17. Procédé selon l'une quelconque des revendications 14-16, où la particule micronisée et le vecteur forment un complexe.

18. Formulation pour l'inhalation obtenue par le procédé selon l'une quelconque des revendications 14-17.
